# EUROPEAN PATENT SPECIFICATION

(11) **EP 3 861 863 B1**
(45) Date of publication and mention of the grant of the patent: **09.10.2024**
(21) Application number: 20156008.3
(22) Date of filing: 07.02.2020
(51) Int. Cl.: A23L 3/32, A61N 1/04, A61N 1/32, A61N 1/40, C12M 1/42, C12N 13/00

(54) **CONDUCTIVITY-ADJUSTED DEVICE FOR TREATING CELLS**
LEITFÄHIGKEITSANGEPASSTE VORRICHTUNG ZUR BEHANDLUNG VON ZELLEN
DISPOSITIF À RÉGLAGE DE CONDUCTIVITÉ POUR LE TRAITEMENT DE CELLULES

(43) Date of publication of application: 11.08.2021
(73) Proprietor: Bühler AG, 9240 Uzwil (CH)
(72) Inventor: HUG, Jsmea, 8460 Marthalen (CH); MATHYS, Alexander, 8044 Zürich (CH); BUCHMANN, Leandro, 8408 Winterthur (CH); GEORGET, Erika, 8041 Zürich (CH)
(74) Representative: Hepp Wenger Ryffel AG

(56) References cited:
- EP-A2- 0 283 700
- EP-B1- 2 308 969
- WO-A2-03/050546
- WO-A2-2005/032646
- US-A- 5 656 926
- US-A1- 2008 076 144

## Description

The present invention relates to a device for treating cells used in medical, environmental, food applications, and bio-based industries (including yeast, bacteria, microalgae, as well as plant or animal cells and cell tissue production systems, in particular targeting inactivation, the extraction of bioactive compounds, and/or the stimulation of cell growth and/or cellular compounds.

It is known that prokaryotic and eukaryotic cells are influenced by the action of electric fields. Stimulation of cell growth, as well as cell death, inactivation of microorganisms, or specific extraction of cell constituents can occur, depending on the applied electric field strength (e.g. Buchmann L and Mathys A (2019), Perspective on Pulsed Electric Field Treatment in the Bio-based Industry, Front. Bioeng. Biotechnol. 7:265, doi: 10.3389/fbioe.2019.00265) .

EP-2 308 969 B1 describes a PEF (pulsed electric field) method where a cell material suspended in an electrically conductive liquid, the cell material being positioned between two electrodes, by exposure of 1 to 100 electric pulses, such that a voltage increase takes place between the two electrodes of 10% to 90% of a target voltage of the electric pulses within a period of 0.1 to 100 ns, the electric pulses have a pulse duration of 5 ns to 5000 ns, and the electric pulses, upon reaching the target voltage, have an electric field strength of 0.5 kV/cm to 50 kV/cm, showed an accelerated cell proliferation and/or increased cell constituents.

In the device for treating cell material used in EP 2 308 969 B1, an electroporation cuvette having the suspension of cell material described above was continuously pumped through an arrangement of two electrodes and exposed to an electric field in an electrically conductive liquid (paragraph [0020] of EP-2 308 969 B1).

The arrangement of two electrodes which provide said electric pulses between them can be considered as a plate capacitor. Depending on the kind of medium that is arranged between the electrodes, the capacity and thus the resistance of said plate capacitor varies. As a result, a safe and steady operation cannot be ensured, since the load applied onto the material to be treated varies depending on the electric conductivity of the material.

EP0283700 discloses a device according to the preamble of claim 1.

It was the problem of the present invention to provide a device for treating cells with electric field in a safe, controllable and steady manner.

The above problem was solved according to the present invention as defined by the independent claims.

In detail, the present invention is related to a device, comprising a unit for generating and emitting electric pulses, two or more electrodes or plates of a capacitor that are arranged in one electric circuit with said unit, and a treatment space arranged between said electrodes or plates so that the treatment space can be penetrated by the emitted electric pulses and an electric field resulting therefrom, characterized in that at least one additional resistor, preferably an adjustable resistor, is arranged in said electric circuit.

It has been found that when a cell material is brought into the treatment space of the device of the invention, it modifies the electric conductivity, as a material that is brought between the plates of a conventional parallel plate capacitor. As a result, the reactance and thus the impedance of the capacitor in an AC circuit are modified.

In order to ensure safe and controlled application of electric pulses onto the cell material within the treatment space, according to the present invention it has been found that any alteration of conductivity evoked by the cell material in the treatment space has to be compensated by at least one additional resistor to be provided in the electric circuit connecting the unit for generating and emitting electric pulses and the two or more electrodes or plates of a capacitor.

Accordingly, at least one resistor is provided in said electric current that precisely compensates for the modified impedance of the treatment chamber, i.e. the two or more electrodes or plates of a capacitor and the treatment space between them.

Since each cell material may have a different effect on the electric conductivity of the device, according to the present invention the at least one additional resistor is an adjustable resistor (also called variable resistor). Adjustable resistors are generally known in the art. Generally, a variable resistor is a resistor whose electric resistance value can be adjusted. A variable resistor is in essence an electromechanical transducer and normally works by sliding a contact (wiper) over a resistive element.

According to a preferred embodiment of the present invention, said at least one adjustable resistor is selected from the group consisting of a potentiometer, a rheostat or a digital potentiometer.

According to one embodiment of the present invention, said at least one adjustable resistor is in a series connection with respect to said electrodes or plates. According to the physical laws of electromagnetism, in a series connection the individual resistance values of resistors add up to a total resistance value in the electric circuit. In cases where the cell material in the treatment space lowers the impedance of the treatment chamber, the at least one additional resistor raises the total resistance value in the electric circuit such that the total resistance value that would otherwise be present without the cell material in the treatment space is achieved.

According to another embodiment of the present invention, said at least one adjustable resistor is in a parallel connection with respect to said electrodes or plates. According to the physical laws of electromagnetism, in a parallel connection the reciprocal individual resistance values of resistors add up to a total resistance value in the electric circuit. In cases where the cell material in the treatment space increases the impedance of the treatment chamber, the at least one additional resistor lowers the total resistance value in the electric circuit such that the total resistance value that would otherwise be present without the cell material in the treatment space is achieved.

According to the present invention, 1 to 10, preferably 1 to 5 of said adjustable resistors are arranged in said electric circuit. The presence of a plurality of adjustable resistors allows an improved fine-tuning of the total resistance value in the electric circuit.

Said at least one adjustable resistor should be provided in the electric circuit between said unit for generating and emitting electric pulses and two or more electrodes or plates of a capacitor.

Electric circuits suitable for the present invention are known to the skilled person. A skilled person can adapt such electric circuits in a routine manner, depending on specific needs to be met by the device of the present invention.

By carefully adjusting the resistance value of the at least one adjustable resistor, matched load conditions can be applied onto the treatment space. The device of the present invention provides for a safe and controlled treatment in the treatment chamber, independent from the electric conductivity of the cell material to be treated.

According to the present invention, said adjustment of the resistance value of said at least one adjustable resistor is performed automatically.

The electric conductivity of the material to be treated is determined before the treatment step.

According to the present invention a unit for measuring the electric conductivity of a material to be treated in the treatment space is provided to generate data for adjusting said at least one adjustable resistor. Such units for measuring the electric conductivity of a material are known in the art and need not be discussed in detail here. Any conventional unit for measuring the electric conductivity of a material can be used for the purposes of the present invention.

In the automatic adjustment of the resistance value of said at least one adjustable resistor, said adjustment is performed, based on the determined value of the electric conductivity of the material to be treated, without interference of user. For example, the unit for measuring the electric conductivity of a material to be treated in the treatment space generates data reflecting the electric conductivity of the material to be treated and transmits them to a component that automatically adjusts the resistance value of said at least one adjustable resistor (e.g. by moving a slider over a contact or in the case of a digital resistor by electronic signals). Alternatively, known data of an electric conductivity of the material to be treated may be provided as input by a user, e.g. via a user terminal, and processed in the device in a manner similar to the data generated by a unit for measuring the electric conductivity. Devices for electrical treatment of cell material are generally known in the art, e.g. from EP-2 308 969 B1 cited above. Preferably, a device as described in one of applicant's copending applications "Device for treating cells", "Non-contact device for treating cells", or "Modified treatment chamber for treating cells" may be used.

Thus, according to one preferred embodiment, a device is used, comprising a unit for generating and emitting electric pulses, a treatment space capable of being penetrated by the emitted electric pulses and an electric field resulting therefrom, wherein the device further comprises a unit which can be moved into and out of said treatment space such that cell material provided within a compartment of said unit, or provided in a container on said unit, can be moved into and out of said treatment space.

Thus, by movement of a unit, a compartment in said unit or a container provided on said unit is moved into a certain region of the device, i.e. the treatment space. Once the respective compartment or container has been moved into the treatment space, an electric field acts on the cell material provided in said compartment or container. Preferably, said movement of the unit is a rotational movement. As a result, the cell material is subjected to the electric field from a uniaxial direction, and a homogeneous stimulation of cell growth is achieved.

The cell material or a suspension containing the cell material is passed through an inlet into said compartment or container which is located inside the treatment unit. The inlet is preferably connected to a device in which the cell material or a suspension containing the cell material is located. For example, conventional pipelines can be provided for this connection.

According to the present invention, conventional containers can be used for receiving the cell material. Examples would be bottles, flasks, test tubes, or cuvettes. Preferably, compartments provided in the unit are of a cylindrical shape. However, also other forms of compartments may be used.

The unit for moving the compartments may be a body, preferably a cylindrical body, which has at least one compartment for receiving the cell material and is arranged on a motor for generating a rotational movement. The compartments (preferably 1 to 20, more preferably 2 to 15, even more preferably 5 to 10 compartments) are preferably blind holes having suitable dimensions.

The device may furthermore comprise filling units and emptying units for filling or emptying said compartments.

Alternatively, the unit may be a plate arranged on a motor for generating a rotational movement, on which plate the at least one container can be fastened. Such units are known. To prevent that the container does not fall off the unit during the, preferably rotational, movement, the unit has means for fastening one or preferably a plurality of containers. Such means are known. They can, for example, be depressions (1 to 20, preferably 2 to 15, more preferably 5 to 10) in the surface of the unit or fastening devices provided on the surface of the unit, such as clamps or closures (screw caps, elastic closures, magnetic closures or the like).

According to another embodiment of the present invention, the device comprises a unit for generating and emitting electric pulses, and a treatment unit having an inlet and an outlet, a treatment space being formed inside the treatment unit, the treatment space being able to be penetrated by the emitted electric pulses and an electric field resulting therefrom, characterized in that a cell material entering through the inlet into the treatment space can be moved without contact through the treatment space and the electric pulses penetrating the treatment space to the outlet.

A non-contact movement (i.e. "without contact") is understood to mean that the material undergoing movement does not come into direct contact with boundary walls (for example, a pipe or a container) during the movement. A non-contact movement according to the present invention is therefore characterized in that there are no interactions with boundary walls during the interaction and in particular no inhomogeneous flow profile (in the case of the movement of a liquid, suspension or emulsion) is formed.

The cell material or a suspension containing the cell material is passed through the inlet of a treatment unit into a treatment space which is located inside the treatment unit. The inlet of the treatment unit is preferably connected to a device in which the cell material or a suspension containing the cell material is located. For example, conventional pipelines can be provided for this connection.

The inlet may comprise a nozzle or the inlet constitutes a nozzle. It is possible to use nozzles which can conventionally be used for introducing a medium into a container or defined space. Such nozzles are known. With this embodiment, the speed at which the cell material or a suspension containing the cell material is introduced into the treatment unit can be adjusted in a targeted manner. The residence time of the cell material in the electric field applied to the treatment space of the treatment unit can thereby be adjusted.

The treatment unit can have any geometric shape which allows a non-contact passage of the cell material. Preferably, the treatment unit is a cylindrical body. This embodiment is characterized by a non-contact passage of the cell material through the treatment space and the electric field penetrating the treatment space to an outlet of the treatment unit. The treatment space therefore has a suitable size to allow the desired non-contact passage of the cell material. According to a preferred embodiment, the treatment unit is a hollow body, particularly preferably a cylindrical hollow body, the interior of which constitutes the treatment space.

Preferably, the passage of the cell material through the treatment space takes place under the action of the gravitational force. In this case, the inlet and outlet are to be arranged relative to one another such that the cell material entering through the inlet passes to the outlet through the action of the gravitational force and can escape therefrom out of the treatment unit. Particularly preferred is an arrangement of inlet and outlet relative to each other such that the outlet is arranged below the inlet, so that the outlet is in the fall line of the inlet. In this case, the inlet is preferably arranged in the top surface, preferably the center of the top surface, of the treatment unit and the outlet arranged in the bottom, preferably the center of the bottom, of the treatment unit. Particularly preferred is thus an arrangement of inlet and outlet relative to each other, in which the outlet is located in the fall line of the inlet. According to the invention, the fall line is the path which a body travels in free fall under the influence of the gravitational force.

Optionally, a receiving device is provided in the treatment unit, with which receiving device the cell material moved through the treatment space can be supplied to the outlet. The receiving device provides an opening which is widened in relation to the opening of the outlet, by means of which cell material not moved on an optimally defined path can reliably be guided to the outlet. For this purpose, the receiving device has an upper opening with a larger area and a lower opening with a smaller area, wherein the size of the areas is related to the respective other opening. According to the invention, the receiving device is preferably a funnel-shaped unit having circular openings.

Preferably, the treatment space is filled with a dielectric material in order to ensure or improve the penetration of the treatment space with the applied electric field. Preferably, the dielectric material is a gas, more preferably air.

Units for generating and emitting electric pulses are well known. According to the present invention, devices which can generate electrical impulses as described below are preferred. Such devices are known. By way of example, cable pulse generators, semiconductor-based pulse generators, or relaxation oscillators can be mentioned.

The generated electric pulses are emitted in a treatment space. This is preferably done by two or more electrodes or plates of a capacitor arranged parallel to each other, which are arranged opposite each another having a distance suitable for the generation of electric pulses. Such arrangements are well known and need not be explained in detail here. Preferably, said two or more electrodes or plates of a capacitor are arranged perpendicularly to the direction of movement of the unit in or on which the compartment or container is provided. When the compartment or container enters the treatment space, it takes a position in the space between said electrodes or plates, so that one electrode/plate is provided over said compartment or container, and the other electrode or plate is provided below said compartment or container.

The electric field to be applied to the treatment space must be characterized such that it provides for the desired effect, e.g. that it stimulates the growth of the treated cells. Corresponding electric fields are known from the prior art, for example from EP-2 308 969 B1. According to the present invention, an electric field generated from such electric pulses can be used, such that a voltage increase takes place between the two electrodes or plates of a capacitor of the device of 10% to 90% of a target voltage of the electric pulses within a period of 0.1 to 1000 ns, the electric pulses have a pulse duration of 5 ns to 50000 ns, and the electric pulses, upon reaching the target voltage, have an electric field strength of 0.5 kV/cm to 100 kV/cm.

According to another embodiment of the present invention, the device comprises a unit for generating and emitting electric pulses, and a treatment unit having an inlet and an outlet, and a treatment space being formed inside the treatment unit such that it can be penetrated by electric pulses and an electric field resulting therefrom, said treatment space being fluidly connected with the inlet and the outlet, characterized in that at least a portion of an inner side face of the treatment space is curved.

A treatment space is provided within the treatment unit, through which treatment space the cell material passes and is thereby treated with electric pulses.

The treatment space can have any geometric shape, but preferably has a cuboid shape. According to a very preferred embodiment of the present invention, said treatment space is positioned in the centre of the treatment unit. Especially preferred the treatment unit is a solid body, and the treatment space is a cavity within said treatment unit, preferably in the centre of the treatment unit. The treatment space within the treatment unit is fluidly connected with the inlet and the outlet of the treatment unit.

At least a portion of an inner side face of the treatment space is curved. In other words, the treatment space does not exhibit fully plain inner side faces, but rather at least partially side faces that influence the flow characteristics of the material protruding through the treatment space. Especially preferred, said inner side face of the treatment space has a sinusoidal shape, at least over a portion thereof. According to this embodiment, a preferred design of the treatment space is such that two side surface of the treatment space which lie opposite to each other are curved, and preferably have a sinusoidal shape, over at least 50%, preferably at least 75% and most preferably 100% of their respective heights.

According to the present invention, the device described herein is suitable for medical, environmental, food applications, and bio-based industries (including yeast, lactobacilli, algae, and cell tissue production systems, in particular including targeted inactivation, the extraction of bioactive compounds, and the stimulation of cell growth and/or cellular compounds. These applications are described in Buchmann L and Mathys A (2019), Perspective on Pulsed Electric Field Treatment in the Bio-based Industry, Front. Bioeng. Biotechnol. 7:265, doi: 10.3389/fbioe. 2019.00265. According to the present invention, basically any material composed of at least one cell, that is, both eukaryotic and prokaryotic cells, can be used as the cell material to be treated. The cell material can be unicellular or multicellular organisms. Examples would be bacteria, yeasts, microalgae, plant cells, and fungal cells or their spores, mycelia, seeds or seedlings and somatic animal cells. Furthermore, multicellular tissues such as meristems in plants and epithelial or connective tissue in humans or animals can be treated.

The cell material is usually (but not necessarily) isolated, purified and/or sterilized in a known manner before being treated according to the present invention. Preferably, the cell material can already be propagated in a known manner in suitable and known culture media to a desired degree before the treatment according to the present invention.

The cell material is preferably suspended in an electrically conductive liquid prior to the treatment according to the present invention. Electrically conductive liquids are well known. According to the present invention, it is necessary to use electrically conductive liquids which have no adverse effects on cell viability, that is, in particular, are non-toxic. According to the present invention, water is preferably used as the electrically conductive liquid, wherein the water can be adjusted to a desired pH value by means of suitable and known additives. According to the present invention, a pH value in the range of 6.0 to 14.0, preferably 7 to 12 is preferred.

According to the present invention, the suspensions described above can be prepared in a conventional manner and stored until treatment. However, the suspensions can also be provided immediately before the treatment according to the present invention.

The present invention is furthermore related to a method for treating cells for targeted inactivation, the extraction of bioactive compounds, and the stimulation of cell growth and/or cellular compounds, preferably performed in a device as described herein, comprising the steps
a) applying an electric field to a treatment space,
b) introducing cell material into the treatment space,
characterized in that the load of said electric field is adjusted to the cell material to be treated by providing at least one additional resistor, preferably by adjusting at least one adjustable resistor.

The method can be performed as already described above.

As stated above, it is preferred that the cell material is provided as a suspension in an electrically conductive liquid in the at least one container.

As stated above, it is further preferred that an electric field is applied with such electric pulses, so that a voltage increase takes place between the two electrodes or plates of a capacitor of the device of 10% to 90% of a target voltage of the electric pulses within a period of 0.1 to 1000 ns, the electric pulses have a pulse duration of 5 ns to 50000 ns, and the electric pulses, upon reaching the target voltage, have an electric field strength of 0.5 kV/cm to 100 kV/cm.

As stated above, according to the present invention, said step of adjusting said at least one adjustable resistor is carried out before step by determining the electric conductivity of the cell material to be treated and automatically adjusting said at least one adjustable resistor depending on said determined electric conductivity.

As stated above, according to the present invention, said step of determining the electric conductivity of the cell material to be treated is carried out by measuring said electric conductivity with a measuring unit.

As stated above, according to one embodiment of the present invention, the method is preferably carried out as follows. First, the unit is moved such that a respective compartment or container in or on said unit is moved into a filling region, where it is filled with cell material through a filling unit. Alternatively, an already filled container may be provided on said unit. Subsequently, the unit is moved again such that the filled compartment or container enters the treatment space. When the filled compartment or container has been moved into the treatment space, movement of the unit is stopped for a period of treatment of the cell material that is present in the treatment space. The electric conductivity of the material to be treated is determined either before the material enters the treatment space, or when the material has entered the treatment space, but before the electric field is applied. In any event, the load of the electric field is adjusted to the material to be treated, by adjusting the resistance value of said at least one adjustable resistor, as described above.

After the treatment has been performed, the filled compartment or container is moved, by movement of the unit, into an emptying region where the treated cell material is removed from the filled compartment or container, as described above. Alternatively, a filled container can be removed from the unit altogether. The empty container or compartment can be subsequently moved again into the filling region, to start another treatment cycle. Optionally, a sterilization/sanitization step may be performed prior to the start of another treatment cycle. Known sterilization/ sanitization processes such as steam-based processes can be used in accordance with the present invention.

According to another embodiment of the present invention, it is preferred that a non-contact passage of the cell material through the treatment space is performed, and the electric field penetrating the treatment space to an outlet of the treatment unit takes place due to gravitational force.

The present invention further relates to the use of the device according to the present invention described here for medical, environmental, food applications, and bio-based industries (including yeast, lactobacilli, algae, and cell tissue production systems, in particular including targeted inactivation, the extraction of bioactive compounds, and the stimulation of cell growth and/or cellular compounds. These applications are described in Buchmann L and Mathys A (2019), Perspective on Pulsed Electric Field Treatment in the Bio-based Industry, Front. Bioeng. Biotechnol. 7:265, doi: 10.3389/fbioe.2019.00265.

The present invention is explained below by way of non-limiting examples and figures. Shown are:
- Fig. 1: a schematic representation of a first embodiment of the device of the present invention
- Fig. 2: a schematic representation of a second embodiment of the device of the present invention
- Fig. 3: a schematic representation of a third embodiment of the device of the present invention
- Fig. 4a-c: a schematic representation of a fourth embodiment of the device of the present invention
- Fig. 5: a schematic illustration of an electric circuit according to a first example of the present invention
- Fig. 6: a schematic illustration of an electric circuit according to a second example of the present invention
- Fig. 7: a schematic illustration of an electric circuit according to a third example of the present invention
- Fig. 8: a schematic illustration of an electric circuit according to a fourth example of the present invention

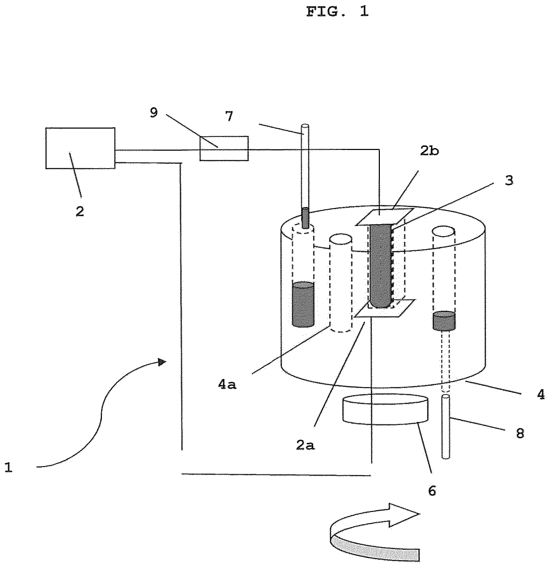

Fig. 1 shows a schematic representation of an embodiment of the device (1) of the present invention.

The device has a unit (2) for generating and emitting electric pulses, for example, a pulse generator. The unit (2) is electrically connected to two electrodes (2a, 2b). A treatment space (3) is located between and closed by the electrodes (2a, 2b), on which treatment space (3) an electric field generated by the unit (2) is applied. The electrodes (2a, 2b) are arranged perpendicularly to the direction of movement of the unit (4). When the compartment (4a) enters the treatment space (3), it takes a position in the space between said electrodes (2a, 2b), so that one electrode (2a) is provided over said compartment (4a), and the other electrode (2b) is provided below said compartment (4a) .

A rotatable unit (4) is provided in the device (1). In the embodiment according to Fig. 1, this unit (4) is a cylindrical body, which by way of example here has 4 compartments (4a) for receiving cell materials. The compartments (4a) are blind holes of suitable dimension.

The rotatable unit (4) is arranged on a motor (6) which generates a rotational movement and sets the unit (4) arranged on it into rotary movement. The compartments (4a) also undergo a rotational movement in this way. Preferably, the unit (4) is rotated at least once around an imaginary axis defined through the center of the unit (4), whereby the compartments (4a) undergo a rotation of 360°. A multiple complete rotation of the unit (4) is possible and advantageous depending on the cell material to be treated.

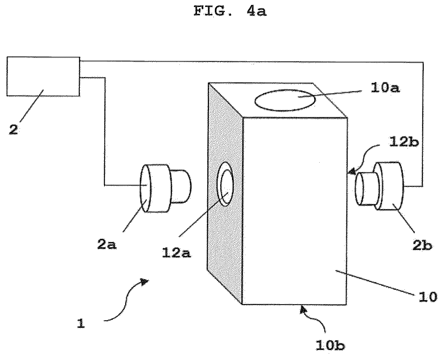

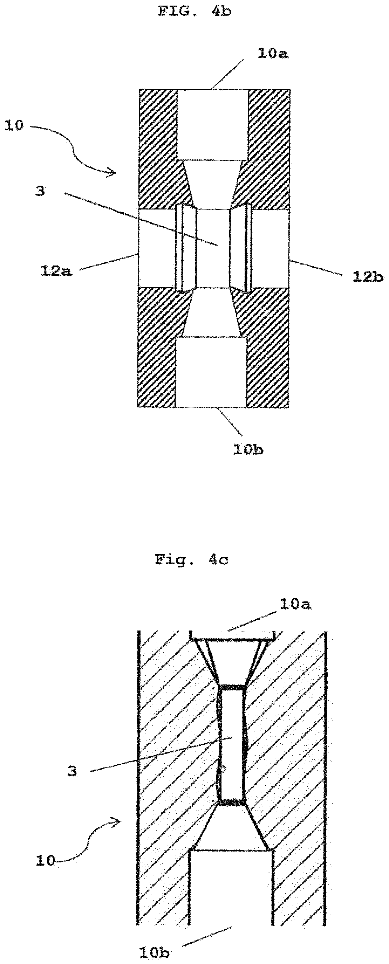

An adjustable resistor (9) is provided between the unit (2) and the electrode/plate (2a). Fig. 1 only shows a schematic illustration of the presence of the adjustable resistor (9). Reference is made to Figs. 4 to 7 for examples of suitable electric circuit arrangements.

In a region of the device (1) preceding the treatment space (3) in the direction of movement of the unit (4), a compartment (4a) is filled with cell material by means of a filling unit (7).

The filling unit (7) is connected to a device (not shown) in which the cell material or a suspension containing the cell material is located.

The filling region is arranged such that the compartment (4a) is first moved into the filling region, where the compartment (4a) is filled to a desired volume (during which time the compartment (4a) remains in a filling position relative to the filling unit (7)), and subsequently the unit (4) is moved further such that the filled compartment (4a) is transferred into the treatment space (3).

Subsequently, the filled compartment (4a) is transported into the treatment space (3), by means of movement of the unit (4). Therein, the cell material in the compartment (4a) undergoes the desired treatment, after the electric field has been adequately adjusted by adjusting the adjustable resistor (9).

After treatment, the cell material is removed from the compartment (4a) by means of an emptying unit (8). In the embodiment of Fig. 1, the emptying unit (8) is a pipeline having an inlet in an emptying region. When the at least one compartment (4a) filled with the treated cell material, after treatment in the treatment space (3), is moved into the emptying region, by means of movement of the unit (4), the compartment (4a) can be connected with the inlet of the emptying unit (8).

The empty compartment (4a) can be subsequently moved again into the filling region, to start another treatment cycle.

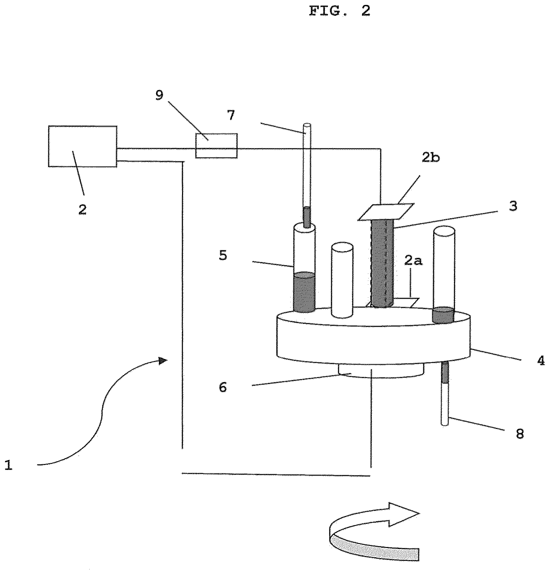

Fig. 2 shows a schematic representation of a second embodiment of the device (1) of the present invention. Like reference numerals designate the same components as in Fig. 1.

In this embodiment, a unit (4) in the form of a plate is provided. Said unit (4) comprises recesses in which one or more containers (5) can be securely arranged so as to not fall off during movement of the unit (4). In the embodiment according to Fig. 2, the unit (4) comprises 4 recesses and thus can take up 4 containers (5).

An adjustable resistor (9) is provided between the unit (2) and the electrode/plate (2a). Fig. 2 only shows a schematic illustration of the presence of the adjustable resistor (9). Reference is made to Figs. 4 to 7 for examples of suitable electric circuit arrangements.

Similar to the embodiment according to Fig. 1, in a region of the device (1) preceding the treatment space (3) in the direction of movement of the unit (4), a container (5) is filled with cell material by means of a filling unit (7).

The filling unit (7) is connected to a device (not shown) in which the cell material or a suspension containing the cell material is located.

The filling region is arranged such that the container (5) is first moved into the filling region, where the container (5) is filled to a desired volume (during which time the container (5) remains in a filling position relative to the filling unit (7)), and subsequently the unit (4) is moved further such that the filled container (5) is transferred into the treatment space (3) .

Alternatively, instead of filling a container (5) in the filling region, also a filled container (5) may be put (manually or automatically) into a free recess on the unit (4).

Subsequently, the filled container (5) is transported into the treatment space (3), by means of movement of the unit (4). Therein, the cell material in the container (5) undergoes the desired treatment, after the electric field has been adequately adjusted by adjusting the adjustable resistor (9).

After treatment, the cell material is removed from the container (5) by means of an emptying unit (8). In the embodiment of Fig. 2, the emptying unit (8) is a pipeline having an inlet in an emptying region. When the at least one container (5) filled with the treated cell material, after treatment in the treatment space (3), is moved into the emptying region, by means of movement of the unit (4), the container (5) can be connected with the inlet of the emptying unit (8).

Alternatively, instead of emptying a container (5) in the emptying region, also a filled container (5) may be removed (manually or automatically) from the unit (4) altogether.

The empty container (5) can be subsequently moved again into the filling region, to start another treatment cycle. Alternatively, an empty or filled container (5) may be put into a free recess on the unit (4).

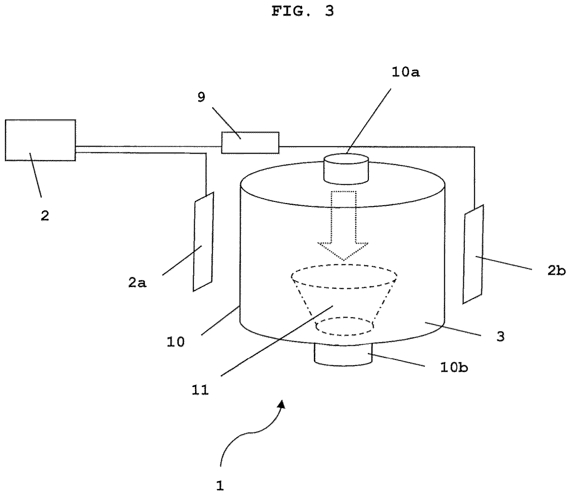

Fig. 3 shows a schematic representation of a further embodiment of the device (1) of the present invention for treating cells for stimulating cell growth.

The device has a unit (2) for generating and emitting electric pulses, for example, a pulse generator. The unit (2) is electrically connected to two electrodes (2a, 2b). A (here cylindrical) treatment unit (10) having a treatment space (3) in its interior is located between the two electrodes (2a, 2b), to which treatment space an electric field generated by the unit (2) is applied, after the electric field has been adequately adjusted by adjusting the adjustable resistor (9).

An adjustable resistor (9) is provided between the unit (2) and the electrode/plate (2b). Fig. 3 only shows a schematic illustration of the presence of the adjustable resistor (9). Reference is made to Figs. 4 to 7 for examples of suitable electric circuit arrangements.

An inlet (10a) is arranged on the top surface of the treatment unit (10) according to this embodiment, preferably in the center of the top surface of the treatment unit (10). An outlet (10b) is arranged on the bottom surface of the treatment unit (10) according to this embodiment, preferably in the center of the bottom surface of the treatment unit (10). According to this embodiment, the outlet (10b) is arranged in the fall line of a cell material which enters into the treatment unit (10) through the inlet (10a) and passes through the treatment space (3) due to gravitational force.

A (here funnel-shaped) receiving device (11) is arranged in the treatment space (3). The receiving device (11) is arranged according to the embodiment shown here in the treatment space (3) immediately above the outlet (10b), so that the lower opening of the receiving device (11) is arranged flush with the (not shown here) opening of the outlet (10b). According to the embodiment shown here, the lower opening of the receiving device (11) and the opening of the outlet (10b) have the same area.

Fig. 4a shows a schematic representation of a further embodiment of the device (1) of the present invention.

The device has a unit (2) for generating and emitting electric pulses, for example, a pulse generator. The unit (2) is electrically connected to two electrodes (2a, 2b). A treatment unit (10) is located between the electrodes (2a, 2b). On a treatment space (3) within said treatment unit (10) (see Fig. 4b and c), electric pulses generated by the unit (2) are applied. The electrodes (2a, 2b) are arranged perpendicularly to the direction of movement of material moving through the treatment unit (10). The material enters the treatment unit (10) through an inlet (10a) in the top face of the treatment unit (10), and leaves the treatment unit (10) through an outlet (10b) in the bottom face of the treatment unit (10).

In two side faces of the treatment unit (10), there are provided openings (12a, 12b), preferably in the form of blind bores, into which the electrodes (2a, 2b) may be inserted and secured.

In Fig 4b, the embodiment of Fig. 4a is shown wherein the treatment unit (10) is a solid body with a treatment space (3) in the form of a cavity in its center. The inlet (10a) in the top face of the treatment unit (10) is fluidly connected, by means of a conduit, with the treatment space (3). In this embodiment, the fluid connection is funnel-shaped over it lower part discharging into the treatment space (3). The outlet (10b) in the bottom face of the treatment unit (10) is also fluidly connected, by means of a conduit, with the treatment space (3). In this embodiment, the fluid connection is funnel-shaped over it upper part extending from the treatment space (3). In two side faces of the treatment unit (10), there are provided openings (12a, 12b) in the form of blind bores for insertion of electrodes. These openings are tapered in a region adjacent to the treatment space (3), i.e. they become smaller as they approach the treatment space (3). The side faces of the treatment space (3) adjacent to the openings (12a, 12b) are plain, i.e. they are not curved.

Fig. 4c is another schematic representation of the treatment unit (3) of the embodiment of the device (1) according to Fig. 4a. As compared to Fig. 4b, the treatment unit (10) is turned by 90°. Thus, in Fig. 4c the side faces of the treatment space (3) are shown that are not adjacent to the openings (12a, 12b). These side faces are curved. Here, they have a sinusoidal shape over their entire height.

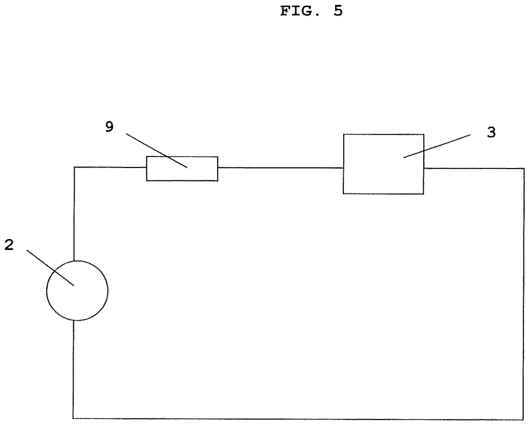

Fig. 5 shows one example of an electric circuit arrangement suitable for the present invention, for example in connection with any of the embodiments shown in Figs. 1 to 4a-c above.

In this example, between a unit (2) for generating and emitting electric pulses and a treatment space (3) in which a material is to be treated by an electric field, there is provided an adjustable resistor (9). In this example, the adjustable resistor (9) and the treatment space (3), respectively the electrodes or plates (2a, 2b) around the treatment space (3), are connected in series.

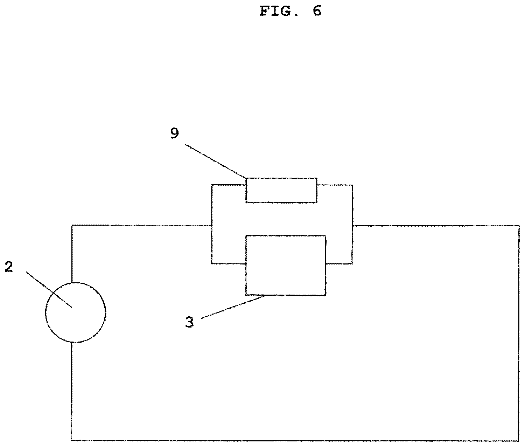

Fig. 6 shows another example of an electric circuit arrangement suitable for the present invention, for example in connection with any of the embodiments shown in Figs. 1 to 4a-c above.

In this example, after a unit (2) for generating and emitting electric pulses, a treatment space (3) in which a material is to be treated by an electric field and an adjustable resistor (9) are provided in a parallel connection.

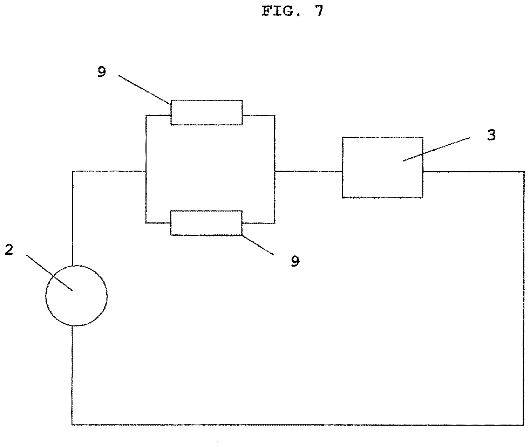

Fig. 7 shows another example of an electric circuit arrangement suitable for the present invention, for example in connection with any of the embodiments shown in Figs. 1 to 4a-c above.

In this example, between a unit (2) for generating and emitting electric pulses and a treatment space (3) in which a material is to be treated by an electric field, there is provided an adjustable resistor (9). In this example, two adjustable resistors (9) are provided in a parallel connection, and these two resistors (9) and the treatment space (3), respectively the electrodes or plates (2a, 2b) around the treatment space (3), are connected in series.

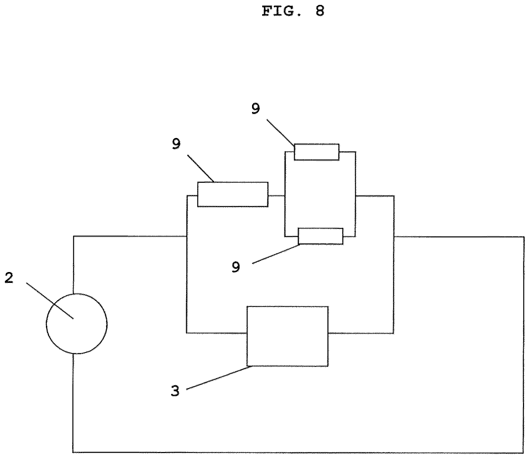

Fig. 8 shows another example of an electric circuit arrangement suitable for the present invention, for example in connection with any of the embodiments shown in Figs. 1 to 4a-c above.

In this example, after a unit (2) for generating and emitting electric pulses, a treatment space (3) in which a material is to be treated by an electric field and three adjustable resistors (9) are provided in a parallel connection. The three adjustable resistors (9) are arranged such that a pair of serially connected adjustable resistors (9) is connected in series to a preceding adjustable resistor (9).

## Claims

1. A device (1), comprising a unit (2) for generating and emitting electric pulses, two or more electrodes or plates (2a, 2b) of a capacitor that are arranged in one electric circuit of said unit (2), and a treatment space (3) arranged between said electrodes or plates (2a, 2b) so that the treatment space (3) can be penetrated by the emitted electric pulses and an electric field resulting therefrom, wherein at least one additional adjustable resistor (9) is arranged in said electric circuit, **characterised in that** a unit for measuring the electric conductivity of a material to be treated in the treatment space (3) is provided to generate data for automatically adjusting said at least one adjustable resistor (9), so as to apply matched load conditions onto the treatment space.

2. The device according to claim 1, **characterized in that** said at least one adjustable resistor (9) is in a series connection with respect to said electrodes or plates (2a, 2b).

3. The device according to claim 1, **characterized in that** said at least one adjustable resistor (9) is in a parallel connection with respect to said electrodes or plates (2a, 2b).

4. The device according to any of claims 1 to 3, **characterized in that** 1 to 10, preferably 1 to 5 of said adjustable resistors (9) are arranged in said electric circuit.

5. The device according to any of claims 1 to 4, **characterized in that** said at least one adjustable resistor (9) is selected from the group consisting of a potentiometer, a rheostat or a digital potentiometer.

6. The device according to any one of claims 1 to 5, **characterized in that** the unit (2) can generate electric pulses, so that a voltage increase take place between the two or more electrodes (2a, 2b) of 10% to 90% of a target voltage of the electric pulses within a period of 0.1 to 1000 ns, the electric pulses have a pulse duration of 5 ns to 50000 ns, and the electric pulses, upon reaching the target voltage, have an electric field strength of 0.5 kV/cm to 100 kV/cm.

7. A method for treating cells for targeted inactivation, the extraction of bioactive compounds, and the stimulation of cell growth and/or cellular compounds, performed in a device (1) according to any one of claims 1 to 6, wherein said method is not performed at a human or animal body, comprising the steps
a) applying an electric field to a treatment space (3),
b) introducing cell material into the treatment space (3),
wherein the load of said electric field is adjusted to the cell material to be treated by adjusting at least one adjustable resistor (9), wherein the step of adjusting said at least one adjustable resistor (9) is carried out before step b) by determining the electric conductivity of the cell material to be treated by a measuring unit and automatically adjusting said at least one adjustable resistor (9) depending on said determined electric conductivity.

8. The method according to claim 7, **characterized in that** determining the electric conductivity of the cell material to be treated is carried out by measuring said electric conductivity with the measuring unit, so as to apply matched load conditions onto the treatment space.

9. The method according to any of claims 7 to 8, **characterized in that** an electric field is applied with such electric pulses, so that a voltage increase takes place between the two electrodes (2a, 2b) or plates of a capacitor of 10% to 90% of a target voltage of the electric pulses within a period of 0.1 to 1000 ns, the electric pulses have a pulse duration of 5 ns to 50000 ns, and the electric pulses, upon reaching the target voltage, have an electric field strength of 0.5 kV/cm to 100 kV/cm.

## Patentansprüche

1. Vorrichtung (1), umfassend eine Einheit (2) zum Erzeugen und Aussenden von elektrischen Impulsen, zwei oder mehr Elektroden oder Platten (2a, 2b) eines Kondensators, die in einem Stromkreis der Einheit (2) angeordnet sind, und einen Behandlungsraum (3), der zwischen den Elektroden oder Platten (2a, 2b) angeordnet ist, so dass der Behandlungsraum (3) von den ausgesendeten elektrischen Impulsen und einem daraus resultierenden elektrischen Feld durchdrungen werden kann, wobei mindestens ein zusätzlicher einstellbarer Widerstand (9) in dem Stromkreis angeordnet ist, **dadurch gekennzeichnet, dass** eine Einheit zur Messung der elektrischen Leitfähigkeit eines zu behandelnden Materials in dem Behandlungsraum (3) bereitgestellt ist, um Daten zur automatischen Einstellung des mindestens einen einstellbaren Widerstands (9) zu erzeugen, um angepasste Lastbedingungen auf den Behandlungsraum anzuwenden.

2. Vorrichtung nach Anspruch 1, **dadurch gekennzeichnet, dass** der mindestens eine einstellbare Widerstand (9) in einer Reihenschaltung zu den Elektroden oder Platten (2a, 2b) steht.

3. Vorrichtung nach Anspruch 1, **dadurch gekennzeichnet, dass** der mindestens eine einstellbare Widerstand (9) in einer Parallelschaltung zu den Elektroden oder Platten (2a, 2b) steht.

4. Vorrichtung nach einem der Ansprüche 1 bis 3, **dadurch gekennzeichnet, dass** 1 bis 10, vorzugsweise 1 bis 5 der einstellbaren Widerstände (9) in dem Stromkreis angeordnet sind.

5. Vorrichtung nach einem der Ansprüche 1 bis 4, **dadurch gekennzeichnet, dass** der mindestens eine einstellbare Widerstand (9) aus der Gruppe bestehend aus einem Potentiometer, einem Rheostat oder einem digitalen Potentiometer ausgewählt ist.

6. Vorrichtung nach einem der Ansprüche 1 bis 5, **dadurch gekennzeichnet, dass** die Einheit (2) elektrische Impulse erzeugen kann, so dass ein Spannungsanstieg zwischen den zwei oder mehr Elektroden (2a, 2b) von 10 % bis 90 % einer Zielspannung der elektrischen Impulse innerhalb einer Zeitspanne von 0,1 bis 1000 ns erfolgt, die elektrischen Impulse eine Impulsdauer von 5 ns bis 50000 ns aufweisen, und die elektrischen Impulse bei Erreichen der Zielspannung eine elektrische Feldstärke von 0,5 kV/cm bis 100 kV/cm aufweisen.

7. Verfahren zur Behandlung von Zellen zur gezielten Inaktivierung, zur Extraktion bioaktiver Verbindungen und zur Stimulierung des Zellwachstums und/oder zellulärer Verbindungen, durchgeführt in einer Vorrichtung (1) nach einem der Ansprüche 1 bis 6, wobei das Verfahren nicht an einem menschlichen oder tierischen Körper durchgeführt wird, umfassend die Schritte
a) Anlegen eines elektrischen Feldes an einen Behandlungsraum (3),
b) Einbringen von Zellmaterial in den Behandlungsraum (3),
wobei die Last des elektrischen Feldes durch Einstellen mindestens eines einstellbaren Widerstandes (9) an das zu behandelnde Zellmaterial angepasst wird, wobei der Schritt des Einstellens des mindestens einen einstellbaren Widerstandes (9) vor Schritt b) durch Bestimmen der elektrischen Leitfähigkeit des zu behandelnden Zellmaterials durch eine Messeinheit und automatisches Einstellen des mindestens einen einstellbaren Widerstandes (9) in Abhängigkeit von der bestimmten elektrischen Leitfähigkeit ausgeführt wird.

8. Verfahren nach Anspruch 7, **dadurch gekennzeichnet, dass** die Bestimmung der elektrischen Leitfähigkeit des zu behandelnden Zellmaterials durch Messung der elektrischen Leitfähigkeit mit der Messeinheit erfolgt, um angepasste Belastungsbedingungen auf den Behandlungsraum anzuwenden.

9. Verfahren nach einem der Ansprüche 7 bis 8, **dadurch gekennzeichnet, dass** ein elektrisches Feld mit derartigen elektrischen Impulsen angelegt wird, dass zwischen den beiden Elektroden (2a, 2b) oder Platten eines Kondensators ein Spannungsanstieg von 10 % bis 90 % einer Zielspannung der elektrischen Impulse innerhalb einer Zeitspanne von 0,1 bis 1000 ns erfolgt, die elektrischen Impulse eine Impulsdauer von 5 ns bis 50000 ns aufweisen, und die elektrischen Impulse bei Erreichen der Zielspannung eine elektrische Feldstärke von 0,5 kV/cm bis 100 kV/cm aufweisen.

## Revendications

1. Dispositif (1) comprenant une unité (2) pour générer et émettre des impulsions électriques, deux électrodes ou plaques (2a, 2b) d'un condensateur ou plus qui sont disposées dans un circuit électrique de ladite unité (2), et un espace de traitement (3) disposé entre lesdites électrodes ou plaques (2a, 2b) de sorte que l'espace de traitement (3) puisse être pénétré par les impulsions électriques émises et un champ électrique qui en résulte, dans lequel au moins une résistance réglable supplémentaire (9) est disposée dans ledit circuit électrique, **caractérisé en ce qu'**une unité de mesure de la conductivité électrique d'un matériau à traiter dans l'espace de traitement (3) est prévue pour générer des données permettant d'ajuster automatiquement ladite au moins une résistance réglable (9), de manière à appliquer des conditions de charge adaptées à l'espace de traitement.

2. Le dispositif selon la revendication 1, **caractérisé en ce que** ladite au moins une résistance réglable (9) est en connexion série par rapport aux électrodes ou plaques (2a, 2b) .

3. Le dispositif selon la revendication 1, **caractérisé en ce que** ladite au moins une résistance réglable (9) est en connexion parallèle par rapport aux électrodes ou plaques (2a, 2b) .

4. Le dispositif selon l'une des revendications 1 à 3, **caractérisé en ce que** 1 à 10, de préférence 1 à 5 desdites résistances réglables (9) sont disposées dans ledit circuit électrique.

5. Le dispositif selon l'une des revendications 1 à 4, **caractérisé en ce que** ladite au moins une résistance réglable (9) est choisie dans le groupe constitué d'un potentiomètre, d'un rhéostat ou d'un potentiomètre numérique.

6. Le dispositif selon l'une des revendications 1 à 5, **caractérisé en ce que** l'unité (2) peut générer des impulsions électriques, de sorte qu'une augmentation de voltage se produise entre les deux électrodes ou plus (2a, 2b) de 10% à 90% d'un voltage cible des impulsions électriques dans une période de 0,1 à 1000 ns, les impulsions électriques ont une durée d'impulsion de 5 ns à 50000 ns, et les impulsions électriques, en atteignant le voltage cible, ont une intensité de champ électrique de 0,5 kV/cm à 100 kV/cm.

7. Méthode de traitement des cellules pour l'inactivation ciblée, l'extraction de composés bioactifs et la stimulation de la croissance cellulaire et/ou de composés cellulaires, réalisée dans un dispositif (1) selon l'une quelconque des revendications 1 à 6, dans laquelle cette méthode n'est pas réalisée sur un corps humain ou animal, comprenant les étapes suivantes
a) appliquer un champ électrique à un espace de traitement (3),
b) introduire du matériel cellulaire dans l'espace de traitement (3),
dans lequel la charge dudit champ électrique est ajustée au matériau cellulaire à traiter en ajustant au moins une résistance réglable (9), l'étape d'ajustement de ladite au moins une résistance réglable (9) étant effectuée avant l'étape b) en déterminant la conductivité électrique du matériau cellulaire à traiter par une unité de mesure et en ajustant automatiquement ladite au moins une résistance réglable (9) en fonction de ladite conductivité électrique déterminée.

8. Méthode selon la revendication 7, **caractérisée par le fait que** la détermination de la conductivité électrique du matériau cellulaire à traiter est effectuée en mesurant ladite conductivité électrique à l'aide de l'unité de mesure, de manière à appliquer des conditions de charge adaptées à l'espace de traitement.

9. La méthode selon l'une des revendications 7 à 8, **caractérisée en ce qu'**un champ électrique est appliqué avec de telles impulsions électriques, de sorte qu'une augmentation de voltage se produit entre les deux électrodes (2a, 2b) ou les plaques d'un condensateur de 10% à 90% d'un voltage cible des impulsions électriques dans une période de 0,1 à 1000 ns, les impulsions électriques ont une durée d'impulsion de 5 ns à 50000 ns, et les impulsions électriques, lorsqu'elles atteignent le voltage cible, ont une intensité de champ électrique de 0,5 kV/cm à 100 kV/cm.
